# EUROPEAN PATENT APPLICATION

(11) **EP 4 835 479 A1**
(43) Date of publication of application: **04.11.2026**
(21) Application number: 24913610.2
(22) Date of filing: 17.12.2024
(51) Int. Cl.: G01N 33/68, G01N 33/558

(54) **BIOMARKER FOR DIAGNOSIS OR PROGNOSTIC EVALUATION OF PERIODONTITIS**

(30) Priority: 29.12.2023 KR 20230196282; 28.11.2024 KR 20240174079
(71) Applicant: Seoul National University R&DB Foundation, Seoul 08826 (KR); UIF (University Industry Foundation), Yonsei University, Seoul 03722 (KR)
(72) Inventor: KIM, Jin Man, Seoul 05268 (KR); LEE, Seung Cheol, Seoul 07801 (KR); CHA, Jae Kook, Seoul 06074 (KR)
(74) Representative: Wilding, Charlotte Katherine
(86) International application number: PCT/KR2024/020485
(87) International publication number: WO 2025/143667

(57) **Abstract**

The present disclosure relates to a biomarker for use in the diagnosis or prognostic evaluation of periodontitis, particularly the grade, stage, severity, and/or aggressiveness of periodontitis, and more specifically, the aggressiveness of periodontitis. The present disclosure can be utilized for the early diagnosis and prediction of aggressive periodontitis, aids in developing personalized treatment strategies for each patient with periodontitis, and may greatly improve the clinical management of aggressive periodontitis.

## Description

### Technical Field

The present invention relates to a biomarker for use in the diagnosis or prognostic evaluation of periodontitis, in particular the grade, stage, severity and/or aggressiveness of periodontitis, particularly the aggressiveness of periodontitis.

### Related Art

Human gingival tissue is composed of various cells encompassing epithelial cells to immune cells, such as epithelial cells, vascular endothelial cells, fibroblasts, B cells, and T cells, and thus has a dynamic ecosystem of cellular interactions. Among them, B cells are major cells which, upon invasion by an external pathogen, develop into plasma cells and produce and secrete antibodies in the immune system. These cells play key roles in the production and maintenance of antibodies and the formation of immune memory, and perform various functions such as immunoglobulin secretion, formation of immune memory, regulation of the immune system, antigen presentation, and T cell activation. Among them, immunoglobulin is a protein that plays an important role in the immune system, and is generally also referred to as an antibody. Immunoglobulins play a key role in adaptive immunity, such as regulating and alleviating immune responses by interacting with various antigens. Immunoglobulins exist in various forms and classes, and the best-known immunoglobulin classes include IgA, IgD, IgE, IgG, and IgM.

Chronic periodontitis is a very commonly occurring periodontal disease, and exhibits inflammatory findings affecting the tissues surrounding the teeth. The gingiva is swollen and red and shows signs of potential bleeding, and when the symptoms are aggravated, chronic inflammation of the gingiva destroys the alveolar bone and causes potential tooth loss. Well-known causes of chronic periodontitis generally include accumulation of oral plaque biofilm infecting the tissues around the teeth and dysbiosis thereof. In the gingiva of patients with chronic periodontitis, high levels of calculus and plaque biofilm accumulation can generally be observed, and this potentially mediates inflammation and stepwise destruction of gingival tissue and adjacent alveolar bone. In the case of chronic periodontitis, since the disease progression is slow and the pathological causal relationship is clear, prevention and treatment strategies for the disease have been well established.

Aggressive periodontitis is a specific form of periodontal disease that rapidly destroys the periodontal ligament and alveolar bone in relatively healthy individuals. Unlike general chronic periodontitis, aggressive periodontitis is known to have little correlation with the amount of oral biofilm and bacterial dysbiosis, and due to various factors, such as onset in relatively healthy and young individuals, early diagnosis and treatment strategies are limited. In particular, strategies such as diagnosing aggressive periodontitis using biomarkers or the like are currently absent.

A disease biomarker is a quantitative or qualitative indicator of a specific molecule, cell, or biological activity measured or detected in a biological sample. Such biomarkers may indicate the occurrence, progression, or prognosis of a specific disease, and are used in various application fields such as diagnosis, prediction, monitoring of therapeutic effects, or understanding of disease mechanisms. Gingival crevicular fluid (GCF) is a small amount of body fluid released from the gingival crevice between the gingiva and the tooth, and reflects information on the condition of the gingiva and periodontal disease.

### SUMMARY OF THE INVENTION

### TECHNICAL PROBLEM

Due to characteristics such as very rapid progression of the disease, serious deterioration in the quality of life of patients caused by tooth loss, and the absence of causes of the disease and appropriate therapeutic strategies, there is an urgent need to develop biomarkers for early diagnosis of aggressive periodontitis, discrimination of high-risk groups, and exploration of the possibility of use as a future therapeutic strategy.

### TECHNICAL SOLUTION

As a result of intensive studies, the inventors of the present invention confirmed that a specific B cell population, which is not found in normal subjects or patients with chronic periodontitis, exists in periodontal tissue of patients with aggressive periodontitis, and, as a result of studying and analyzing the same, first revealed that, in periodontal tissue of patients with aggressive periodontitis, there is a high proportion of B cells that have failed to develop into mature plasma cells due to the absence of a CSR (Class Switch Recombination) process. Based on this, the inventors have been able to provide a method for providing information on the diagnosis or prognostic evaluation of periodontitis, in particular the grade, stage, severity and/or aggressiveness of periodontitis, particularly the aggressiveness of periodontitis, by measuring the level of such immature B cells. The level of immature B cells may preferably be represented by the level of IgM expressed in the corresponding B cells.

Accordingly, the present invention relates to a method for providing information on the diagnosis or prognostic evaluation of periodontitis, the method comprising: measuring information indicating a level of B cells at a stage before being developed into plasma cells in a biological sample isolated from a subject; and providing information on diagnosis or prognostic evaluation of periodontitis from the information. The information on the level of B cells at a stage before being developed into plasma cells in the biological sample isolated from the subject preferably includes, but is not necessarily limited to, an expression level of IgM in the biological sample.

In the present invention, the biological sample may be derived from gingival crevicular fluid of the subject.

In the present invention, the periodontitis may be aggressive periodontitis.

In the present invention, the periodontitis may be chronic periodontitis.

In the present invention, the periodontitis may be Grade C periodontitis.

In the present invention, the periodontitis may be Grade B periodontitis.

In the present invention, the periodontitis may be Grade A periodontitis.

In the present invention, the level of B cells at a stage before being developed into plasma cells in the biological sample isolated from the subject is higher than a reference level may indicate one or more of the following states:
i) the subject has a rapid progression rate of periodontitis or has severe periodontitis;
ii) the subject has a high risk of being diagnosed with Grade B periodontitis, Grade C periodontitis, or aggressive periodontitis;
iii) the subject has a high risk of being diagnosed with stage 3 or stage 4 periodontitis; and
iv) the subject has a high risk of being diagnosed with IgM-secreting B cell-infiltrated periodontitis.

In the present invention, the level of B cells at a stage before being developed into plasma cells in the biological sample isolated from the subject is lower than a reference level may indicate one or more of the following states:
i) the subject does not have a rapid progression rate of periodontitis and does not have severe periodontitis;
ii) the subject has a low risk of being diagnosed with Grade B periodontitis, Grade C periodontitis, or aggressive periodontitis;
iii) the subject has a low risk of being diagnosed with stage 3 or stage 4 periodontitis; and
iv) the subject has a low risk of being diagnosed with IgM-secreting B cell-infiltrated periodontitis.

In the present invention, the IgM expression level in the biological sample isolated from the subject is higher than a reference level may indicate one or more of the following states:
i) the subject has a rapid progression rate of periodontitis or has severe periodontitis;
ii) the subject has a high risk of being diagnosed with Grade B periodontitis, Grade C periodontitis, or aggressive periodontitis;
iii) the subject has a high risk of being diagnosed with stage 3 or stage 4 periodontitis; and
iv) the subject has a high risk of being diagnosed with IgM-secreting B cell-infiltrated periodontitis.

In the present invention, the IgM expression level in the biological sample isolated from the subject is lower than a reference level may indicate one or more of the following states:
i) the subject does not have a rapid progression rate of periodontitis and does not have severe periodontitis;
ii) the subject has a low risk of being diagnosed with Grade B periodontitis, Grade C periodontitis, or aggressive periodontitis;
iii) the subject has a low risk of being diagnosed with stage 3 or stage 4 periodontitis; and
iv) the subject has a low risk of being diagnosed with IgM-secreting B cell-infiltrated periodontitis.

In the present invention, the reference level may be equivalent to any one measured as follows:
i) measured from a biological sample isolated from a normal subject;
ii) measured from a biological sample isolated from a patient suffering from Grade A periodontitis; or
iii) measured from a biological sample isolated from a patient suffering from Grade B periodontitis.

In the present invention, a method for measuring the expression level of IgM may be selected from the group consisting of Western blotting, immunochemical analysis, flow cytometry, and a protein chip.

In the present invention, the information on diagnosis or prognostic evaluation of periodontitis may be information on a grade, stage, severity and/or aggressiveness of periodontitis.

The present invention also relates to a biomarker composition usable for diagnosing or evaluating a prognosis of periodontitis, the biomarker composition comprising an agent capable of measuring the level of B cells at a stage before being developed into plasma cells, particularly an agent capable of measuring the expression level of IgM.

In the present invention, the agent capable of measuring the IgM expression level may be one or more selected from the group consisting of an antibody, a peptide, an aptamer, and a compound that specifically bind to IgM.

In the present invention, the biological sample may be derived from gingival crevicular fluid of the subject.

In addition, the present invention relates to various diagnostic uses of such a biomarker composition, for example, a kit for diagnosing or evaluating a prognosis or periodontitis using such a biomarker composition. In the present invention, the diagnosing or evaluating a prognosis of periodontitis may relate to the grade, stage, severity and/or aggressiveness of periodontitis.

In the present invention, the kit may be for providing information on a grade, stage, severity and/or aggressiveness of periodontitis.

In the present invention, the expression level of IgM may be represented as a ratio of the expression level of IgM to an expression level of IgG or an expression level of IgA.

In the present invention, the expression level of IgM may be represented as a ratio of the expression level of IgM to an expression level of IgG and IgA.

### ADVANTAGEOUS EFFECTS

The method of the present invention can be utilized for early diagnosis and prediction of aggressive periodontitis, and has an advantage in that it can assist in developing a customized periodontitis treatment strategy for each individual patient, thereby providing a more future-oriented and specialized personalized treatment strategy. In view of the fact that aggressive periodontitis is still a representative unconquered and intractable disease in the field of dentistry, for which diagnostic criteria remain uncertain and effective treatment strategies have not been properly established, the method of the present invention can greatly improve the current treatment situation, which is being carried out in the aspect of managing symptoms only after aggressive periodontitis has worsened, by enabling the risk of aggressive periodontitis to be evaluated early and conveniently, and can accordingly reduce a large amount of social costs caused thereby.

### BRIEF DESCRIPTION OF THE DRAWINGS

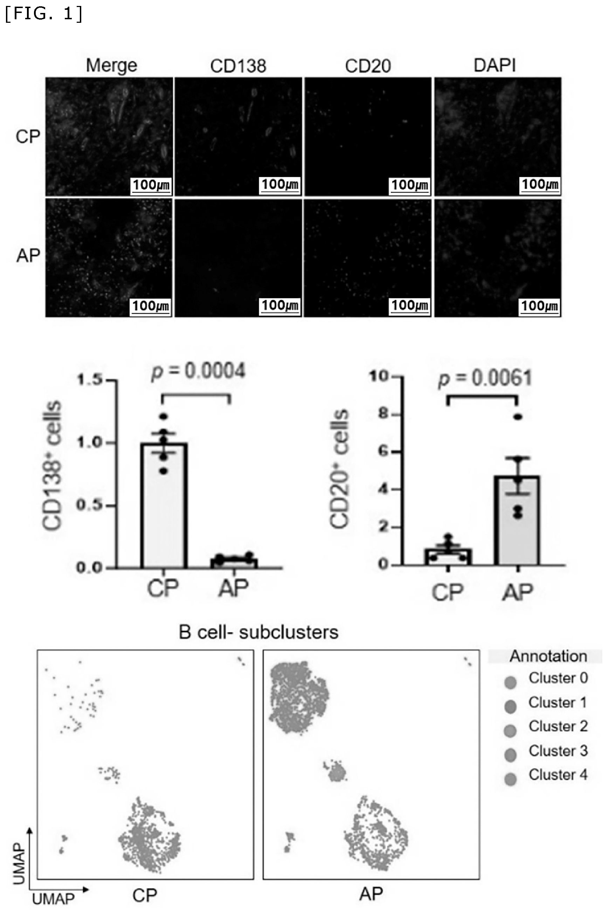

FIG. 1 shows periodontal tissue-specific plasma cell and B cell distributions in patients with aggressive periodontitis compared with chronic periodontitis. As results of predicting a disease-specific CSR (Class Switch Recombination) pattern through analysis of distributions of plasma cells (CD138⁺ cells) and B cells (CD20⁺ cells) in periodontal tissues of patients with aggressive periodontitis and patients with chronic periodontitis, and results of B cell population distribution through single-cell sequencing of gingival tissue, significance was indicated by p values in comparison with a control group (CP) (CP: chronic periodontitis, AP: aggressive periodontitis). In the B cell subcluster distribution map, clusters of dots mainly located at the upper left and upper right correspond to cluster 0, clusters of dots mainly located at the lower right correspond to cluster 1, clusters of dots mainly located at the center correspond to cluster 2, and clusters of dots mainly located at the lower left correspond to cluster 3. Dots corresponding to cluster 4 were found in extremely small amounts in the regions of clusters 1 to 3. It should be noted that the B cell population analysis results of patients with chronic periodontitis and the B cell population analysis results of patients with aggressive periodontitis show significant differences in the overall pattern, for example, the amount of cluster 0.

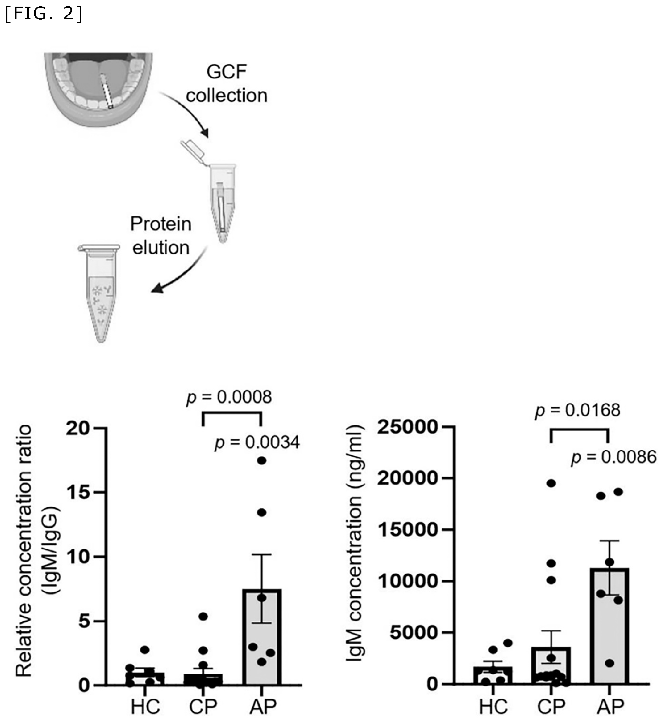

FIG. 2 shows an aggressive periodontitis patient GCF-specific immunoglobulin expression pattern and a ratio thereof. An aggressive periodontitis-specific biomarker was discovered through disease-specific immunoglobulin visualization and ratio quantification by analysis of concentrations of specific immunoglobulins (IgM and IgG) in gingival crevicular fluid, and significance was indicated by p values in comparison with a control group (HC: Healthy, CP: Chronic periodontitis, AP: Aggressive periodontitis, NS: Not significant).

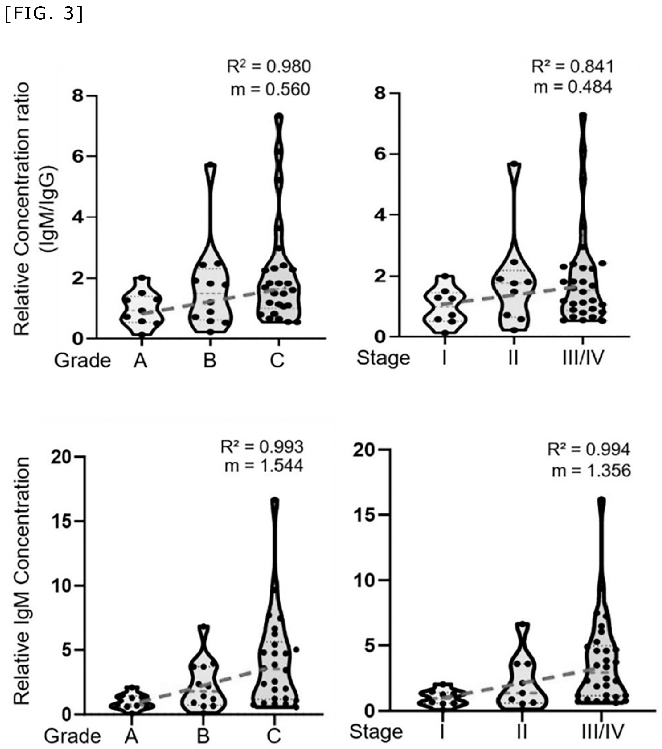

FIG. 3 shows results indicating relevance between the biomarkers discovered in FIG. 2 (IgM/IgG ratio and IgM concentration) and the severity of periodontitis by classifying the grade and stage of periodontitis at an end point of a 5-year retrospective cohort and collecting GCF from patients. An increasing or decreasing tendency was indicated by a slope value (m) with respect to the mean of each y-axis value, and it was confirmed that the IgM/IgG ratio and IgM concentration are closely related to changes in the grade and stage of periodontitis.

### BEST MODE FOR CARRYING OUT THE INVENTION

The present invention provides a method for providing information on the diagnosis or prognostic evaluation of periodontitis, the method comprising: measuring information on a level of B cells at a stage before being developed into plasma cells in a biological sample isolated from a subject; and providing information on diagnosis or prognostic evaluation of periodontitis from the information.

In the present invention, the subject may include, without limitation, mammals such as human, primates including chimpanzees, companion animals such as dogs and cats, livestock animals such as cattle, horses, sheep, and goats, and rodents such as mice and rats.

In the present invention, the biological sample includes biological samples such as tissue, cells, blood, serum, plasma, saliva, and body fluid. However, according to one embodiment of the present invention, the biological sample is preferably derived from gingival crevicular fluid (GCF) of the subject. In the present invention, the gingival crevicular fluid is a body fluid secreted from the gingival crevice, which is a minute gap between the gingiva and the tooth.

B cells express and secrete immunoglobulins on their surface, and, according to the development of B cells, the type of immunoglobulin production that is expressed changes. Such a biological mechanism is referred to as CSR (Class Switch Recombination). Generally, mature B cells serve to express IgM on their surface and secrete the same. Such B cells generally develop into plasma cells that perform a substantial effector function after initial contact with an antigen, and, during the developmental process of such a cell type, switch the antibody production type from the initially expressed IgM antibody production type to IgG, IgA, or IgE. Through such a CSR process, in each tissue, the ratio of IgM secreted by mature B cells and IgG, IgA, IgE, and the like secreted by plasma cells achieves immune homeostasis according to each time point. A representative immunoglobulin type found in gingival crevicular fluid is IgG, which is widely distributed in circulating blood or tissue fluid and is known to be predominantly found in gingival crevicular fluid of inflamed gingiva.

The inventors of the present invention analyzed cells obtained from GCF samples obtained from healthy individuals, chronic periodontitis patients, and aggressive periodontitis patients, and, as a result, identified an immature B cell population specifically appearing in aggressive periodontitis patients. In addition, as a result of analyzing immunoglobulin molecules expressed by B cells, it was observed that, in aggressive periodontitis patients, the expression level of IgM was higher than those of other immunoglobulin molecules. Accordingly, the inventors of the present invention found that an excessively high distribution of immature B cells due to a lack of a CSR process is a distinguishing characteristic exhibited by aggressive periodontitis, and thereby provided a method capable of diagnosing or evaluating a prognosis of periodontitis, particularly a grade, stage, severity and/or aggressiveness of periodontitis, particularly aggressiveness of periodontitis, by measuring the level of such immature B cells.

Information indicating the level of B cells at a stage before being developed into plasma cells may be provided, for example, through single-cell analysis of a sample obtained from a subject to be analyzed, and may also be provided through analysis of expression levels, by type, of proteins specifically expressed on the surface of the B cells, for example, immunoglobulin molecules. A person having ordinary skill in the art of biotechnology is well aware of characteristics of B cells at a stage before being developed into plasma cells and methods for measuring the level of such B cells.

The information indicating the level of B cells at a stage before being developed into plasma cells in a biological sample isolated from a subject may preferably include an expression level of IgM in the biological sample. When B cells develop into plasma cells through a normal CSR process, the antibody production type is switched from an initially expressed IgM antibody production type to IgG, IgA or IgE. Therefore, the fact that the expression level of IgM is higher than a reference level indicates that the level of immature B cells is high. As a result of experiments conducted by the inventors of the present invention, aggressive periodontitis patients exhibited a higher expression level of IgM than healthy individuals or chronic periodontitis patients, and it was confirmed that the expression level was independent of the expression levels of IgG and/or IgA. Therefore, it is possible to diagnose or prognostically evaluate aggressive periodontitis not only by comparing the expression level of IgM with that of an immunoglobulin such as IgG or IgA, but also by using the expression level of IgM alone. In this respect, in various aspects of the present invention, measuring the expression level of IgM may include measuring a ratio of the expression level of IgM to the expression level of IgG, measuring a ratio of the expression level of IgM to the expression level of IgA, or measuring a ratio of the expression level of IgM to the expression levels of IgG and IgM.

In the present invention, the measurement of an expression level of a specific protein, including IgM, may be performed using ordinary techniques in the field of biotechnology for confirming the presence or absence and expression degree of a protein, and a person having ordinary skill in the art is familiar with such ordinary techniques. For example, techniques such as Western blotting, immunochemical analysis, flow cytometry, or a protein chip may be used. The immunochemical analysis refers to a method for confirming the presence or absence and/or amount of a specific protein through an antigen-antibody reaction, and examples thereof include, but are not limited to, ELISA (enzyme-linked immunosorbent assay), RIA (radioimmunoassay), radioimmunodiffusion, Ouchterlony immunodiffusion, rocket immunoelectrophoresis, tissue immunostaining, immunoprecipitation assay, complement fixation assay, and the like.

As used herein, the term "diagnosis" means determining whether a subject currently has periodontitis, or determining a type, for example, chronic periodontitis or aggressive periodontitis, grade, stage, severity, or aggressiveness of periodontitis currently possessed by the subject. As used herein, the term "prognosis" means predicting or preliminarily evaluating whether a subject will have periodontitis in the future, or a type, for example, chronic periodontitis or aggressive periodontitis, grade, stage, severity, or aggressiveness of periodontitis that the subject may have in the future. In this respect, the phrase "diagnosis or prognostic evaluation" as used herein is interpreted as including diagnosing or evaluating a type, grade, stage, severity, or aggressiveness of periodontitis.

The method of the present invention may be used to diagnose chronic periodontitis or aggressive periodontitis, or to evaluate a possibility, that is, a risk, of having chronic periodontitis or aggressive periodontitis. In this respect, "periodontitis" mentioned herein may be chronic periodontitis or aggressive periodontitis.

Criteria for classifying periodontal diseases have changed according to the times and progress of research, and a new classification system was recently proposed in 2018 through the World Workshop, following the classification systems of 1989 and 1999. The 1999 classification system has been widely used to date, and a major characteristic thereof was that adult periodontitis in the 1989 classification was replaced with chronic periodontitis, and early-onset periodontitis was replaced with aggressive periodontitis. Aggressive periodontitis means a destructive periodontal disease in which loss of hard tissues and soft tissues around teeth progresses at a faster rate than in chronic periodontitis, thereby causing a high risk of tooth loss. As used herein, "chronic periodontitis" and "aggressive periodontitis" mean periodontitis having severity and/or aggressiveness equivalent to periodontitis classified, under the 1989 classification system, as adult periodontitis and early-onset periodontitis, respectively, and, under the 1999 classification system, as chronic periodontitis and aggressive periodontitis, respectively.

In 2018, a new classification system for periodontal diseases was introduced. The 2018 classification system classifies patients into a periodontally healthy state, gingivitis, and periodontitis. Periodontitis is classified into necrotizing periodontitis, periodontitis, and periodontitis associated with systemic diseases. Periodontitis includes chronic periodontitis and aggressive periodontitis under the 1999 classification system. A patient diagnosed with periodontitis is finally diagnosed by being classified according to Stage and Grade. Stage reflects the severity/extent of the disease and the complexity of treatment, and Grade reflects the rate of progression of the disease and the risk of further progression. Patients are classified into Stage I to Stage IV according to clinical attachment level or bone loss, and Grade is classified into A, B, and C by reflecting the rate of progression of periodontal disease measured by a bone loss/age index, smoking status, and diabetes. Specifically, Stage I corresponds to initial periodontitis, Stage II corresponds to moderate periodontitis, Stage III corresponds to severe periodontitis with potential for tooth loss, and Stage IV corresponds to severe periodontitis with potential for loss of dentition. Grade A represents a slow rate of progression, Grade B represents a moderate rate of progression, and Grade C represents a rapid rate of progression. As used herein, "chronic periodontitis" may mean periodontitis having severity and/or aggressiveness equivalent to periodontitis classified as Grade A or Grade B under the 2018 classification system. As used herein, "aggressive periodontitis" may mean periodontitis having severity and/or aggressiveness equivalent to periodontitis classified as Grade C under the 2018 classification system.

As used herein, the term "grade" may mean Grade under the 2018 classification system. As used herein, the term "stage" may mean Stage under the 2018 classification system. As used herein, the term "severity" means the degree of symptoms and lesions of a certain disease, and may be classified, for example, into mild, moderate, and severe. The term "severity" may mean Stage under the 2018 classification system. As used herein, the term "aggressiveness" means a rate of progression of periodontitis, and may mean Grade under the 2018 classification system.

In the context of periodontitis under the 2018 classification system, the method of the present invention may be used to diagnose Grade C periodontitis or to evaluate a possibility, that is, a risk, of having Grade C periodontitis. For example, when a subject exhibits a level of immature B cells higher than the level of immature B cells exhibited by a healthy individual or a patient with Grade A or Grade B periodontitis, or exhibits an IgM expression level higher than the IgM expression level exhibited by a healthy individual or a patient with Grade A or Grade B periodontitis, the subject may be diagnosed as having Grade C periodontitis or may be evaluated as having a high possibility, that is, a high risk, of having Grade C periodontitis.

In the context of periodontitis under the 2018 classification system, the method of the present invention may be used to diagnose Grade B periodontitis or to evaluate a possibility, that is, a risk, of having Grade B periodontitis. For example, when a subject exhibits a level of immature B cells lower than the level of immature B cells exhibited by a patient with Grade C periodontitis, or exhibits an IgM expression level lower than the IgM expression level exhibited by a patient with Grade C periodontitis, the subject may be diagnosed as having Grade B periodontitis or may be evaluated as having a high possibility, that is, a high risk, of having Grade B periodontitis. In addition, when a subject exhibits a level of immature B cells higher than the level of immature B cells exhibited by a healthy individual or a patient with Grade A periodontitis, or exhibits an IgM expression level higher than the IgM expression level exhibited by a healthy individual or a patient with Grade A periodontitis, the subject may be diagnosed as having Grade B periodontitis or may be prognostically evaluated as having Grade B periodontitis.

In the context of periodontitis under the 2018 classification system, the method of the present invention may be used to diagnose Grade A periodontitis or to evaluate a possibility (risk) of having Grade A periodontitis. For example, when a subject exhibits a level of immature B cells higher than the level of immature B cells exhibited by a healthy individual, or exhibits an IgM expression level higher than the IgM expression level exhibited by a healthy individual, the subject may be diagnosed as having Grade A periodontitis or may be evaluated as having a high possibility (risk) of having Grade A periodontitis. In addition, when a subject exhibits a level of immature B cells lower than the level of immature B cells exhibited by a patient with Grade B or Grade C periodontitis, or exhibits an IgM expression level lower than the IgM expression level exhibited by a patient with Grade B or Grade C periodontitis, the subject may be diagnosed as having Grade A periodontitis or may be prognostically evaluated for Grade A periodontitis.

The method of the present invention may also be used to diagnose periodontitis under the 2018 classification system or to evaluate a possibility (risk) of having periodontitis. For example, when a subject exhibits a level of immature B cells lower than the level of immature B cells exhibited by a patient with Grade A, Grade B or Grade C periodontitis, or exhibits an IgM expression level lower than the IgM expression level exhibited by a patient with Grade A, Grade B or Grade C periodontitis, the subject may be evaluated as not currently having periodontitis, particularly aggressive periodontitis, or as having a low risk thereof.

The method of the present invention may be useful for providing information regarding a grade, stage, severity and/or aggressiveness of periodontitis. The grade, stage, severity and aggressiveness of periodontitis referred to herein should not be construed as being determined while being limited to a periodontitis classification system at a specific point in time.

Specifically, the method of the present invention may be utilized, based on the fact that the level of B cells at a stage before being developed into plasma cells in the biological sample isolated from the subject is higher than a reference level, as indicating one or more of the following states:
i) the subject has a rapid progression rate of periodontitis or has severe periodontitis;
ii) the subject has a high risk of being diagnosed with Grade B periodontitis, Grade C periodontitis, or aggressive periodontitis;
iii) the subject has a high risk of being diagnosed with Stage 3 or Stage 4 periodontitis; and
iv) the subject has a high risk of being diagnosed with IgM-secreting B cell-infiltrated periodontitis.

In another manner, the method of the present invention may be utilized, based on the fact that the level of B cells at a stage before being developed into plasma cells in the biological sample isolated from the subject is lower than a reference level, as indicating one or more of the following states:
i) the subject does not have a rapid progression rate of periodontitis and does not have severe periodontitis;
ii) the subject has a low risk of being diagnosed with Grade B periodontitis, Grade C periodontitis, or aggressive periodontitis;
iii) the subject has a low risk of being diagnosed with Stage 3 or Stage 4 periodontitis; and
iv) the subject has a low risk of being diagnosed with IgM-secreting B cell-infiltrated periodontitis.

In another manner, the method of the present invention may be utilized, based on the fact that the IgM expression level in a biological sample isolated from a subject is higher than a reference level, as indicating one or more of the following states:
i) the subject has a rapid progression rate of periodontitis or has severe periodontitis;
ii) the subject has a high risk of being diagnosed with Grade B periodontitis, Grade C periodontitis, or aggressive periodontitis;
iii) the subject has a high risk of being diagnosed with Stage 3 or Stage 4 periodontitis; and
iv) the subject has a high risk of being diagnosed with IgM-secreting B cell-infiltrated periodontitis.

In another manner, the method of the present invention may be utilized, based on the fact that the IgM expression level in the biological sample isolated from the subject is lower than a reference level, as indicating one or more of the following states:
i) the subject does not have a rapid progression rate of periodontitis and does not have severe periodontitis;
ii) the subject has a low risk of being diagnosed with Grade B periodontitis, Grade C periodontitis, or aggressive periodontitis;
iii) the subject has a low risk of being diagnosed with Stage 3 or Stage 4 periodontitis; and
iv) the subject has a low risk of being diagnosed with IgM-secreting B cell-infiltrated periodontitis.

In the present invention, the term "IgM-secreting B cell-infiltrated periodontitis" means any periodontitis characterized in that B cells characterized by IgM oversecretion are increased in periodontal tissue.

As used herein, the term "reference level" refers to a value measured from an individual or population of individuals whose grade, stage, severity and/or aggressiveness of periodontitis is potentially the same as or different from that of a subject to be provided with information regarding diagnosis or prognostic evaluation of periodontitis according to the present invention, or a value set as being equivalent to such measured value, and is for comparison with a value measured from the subject. Specifically, such reference level may be, according to various purposes that may be set based on the technical idea of the present invention, a value representing the level of immature B cells, that is, B cells at a stage before being developed into plasma cells, an IgM expression level, or a ratio of IgM to another immunoglobulin, for example, IgG or IgA, or another protein in gingival crevicular fluid, measured from a healthy individual, a chronic periodontitis patient, an aggressive periodontitis patient, a Grade A periodontitis patient, a Grade B periodontitis patient, a Grade C periodontitis patient, a Stage I patient, a Stage II patient, or a Stage III patient.

As used herein, the term "reference level" may be, for example, equivalent to any one of the following measured values: i) a value measured from a biological sample isolated from a healthy individual; ii) a value measured from a biological sample isolated from a patient suffering from chronic periodontitis under the 1999 classification system, or periodontitis equivalent thereto; iii) a value measured from a biological sample isolated from a patient with Grade A periodontitis under the 2018 classification system, or periodontitis equivalent thereto; or iv) a value measured from a biological sample isolated from a patient with Grade B periodontitis under the 2018 classification system, or periodontitis equivalent thereto.

In addition, the present invention provides a biomarker composition for diagnosing or evaluating a prognosis of periodontitis, the biomarker composition comprising an agent for measuring the level of B cells at a stage before being developed into plasma cells in a biological sample isolated from a subject. Preferably, the agent is an agent capable of measuring an IgM expression level in a biological sample isolated from a subject.

In the present invention, the agent capable of measuring the IgM expression level may be one or more selected from the group consisting of an antibody, a peptide, an aptamer and a compound that specifically bind to IgM.

In the present invention, the term "antibody" is a term known in the art and means a specific protein molecule directed against an antigenic site. For the purpose of the present invention, the antibody means an antibody that specifically binds to IgM of the present invention, and such antibody may be prepared from the IgM by a conventional method by cloning each gene into an expression vector according to a conventional method, or a commercially available anti-IgM antibody may be used.

The form of the antibody is not particularly limited, and a polyclonal antibody, a monoclonal antibody, or a portion thereof is also included in the antibody of the present invention as long as it has antigen-binding ability, and all immunoglobulin antibodies are included. Furthermore, the antibody of the present invention also includes special antibodies such as humanized antibodies. The antibody against IgM of the present invention may be any antibody that can be prepared by methods known in the art. For example, the antibody of the present invention may include not only a complete form having two full-length light chains and two full-length heavy chains, but also a functional fragment of an antibody molecule. The functional fragment of an antibody molecule means a fragment retaining at least an antigen-binding function, and may be Fab, F(ab'), F(ab')2, Fv, or the like, but is not limited thereto.

In the present invention, the measurement includes quantitative and/or qualitative analysis, and includes measurement of presence or absence and measurement of an expression amount. Such methods are known in the art, and a person skilled in the art would be able to select an appropriate method for carrying out the present invention.

Meanwhile, among the components of the biomarker composition, descriptions of technical features overlapping with the method for providing information regarding diagnosis or prognostic evaluation of periodontitis are omitted.

In addition, the present invention provides a kit comprising the biomarker composition. The kit is useful for diagnosis or prognostic evaluation of periodontitis, particularly a grade, stage, severity and/or aggressiveness of periodontitis, particularly aggressiveness of periodontitis.

In the present invention, the kit may include not only an antibody recognizing IgM, but also a composition, solution, or device comprising one or more other components suitable for an analysis method. The kit may further include, in addition to the antibody recognizing IgM, an antibody molecule against another immunoglobulin such as IgG or IgA.

Based on the contents described above, the present invention may relate to, but is not limited to, a method for providing information regarding diagnosis or prognostic evaluation of periodontitis, a biomarker composition, and a kit as described below.

Hereinafter, the present invention will be described in detail with reference to examples in order to aid understanding of the present invention. However, the following examples are merely illustrative of the contents of the present invention, and the scope of the present invention is not limited to the following examples. The examples of the present invention are provided to more completely explain the present invention to those having ordinary skill in the art.

### Example 1. Materials and Methods

### 1.1. Collection of Gingival Crevicular Fluid (GCF)

In the present invention, GCF of each patient was collected by inserting prefabricated paper points (#20) having the same specification into a gingival crevice for 20 seconds. The collected samples (paperpoint, 3 ea) were placed in 200 µl of a solvent (DPBS + 0.1% Tween 20) and treated using a tabletop vortexer for 30 minutes so as to allow the samples to be eluted into the solvent, thereby obtaining samples in a form suitable for ELISA measurement.

### 1.2. ELISA Analysis

The ELISA technique used to derive the results of the present invention is a method of immunocapturing an analyte contained in a sample, namely IgM and IgG in the present invention, using a capture antibody labeled with an affinity tag and a detection antibody conjugated with a reporter. The entire complex to be analyzed, i.e., the capture antibody, the analyte, and the detection antibody, was immobilized in each well based on an immunoaffinity principle. In the present invention, measurement was performed according to the manufacturer's protocol using SimpleStep ELISA kits manufactured by Abcam (IgM: #214568, Abcam, UK; IgG: #195215, Abcam, UK), and a biomarker was specified through quantification of the ratio thereof. The detailed method is as follows.

First, a sample and a standard, which is a standard material for standardizing a sample concentration, were added to a well in an amount of 50 µL. In the case of the sample, it is necessary to previously set up a concentration suitable for analysis through a serial dilution method. Then, 50 µL of an antibody mixture was added thereon. Thereafter, incubation was performed for a time specified in the protocol of each SimpleStep ELISA kit. After incubation, each well was washed to remove unbound materials. Thereafter, a TMB development solution (100 µL) was added, and, incubation, the TMB development solution was catalyzed by HRP, thereby causing the sample to turn blue. Meanwhile, the reaction was stopped by adding a stop solution before saturation, whereby a color change from blue to yellow could be observed. The color change proceeds in proportion to the amount of the bound analyte. The absorbance was measured at 450 nm using a well-based absorbance measuring device, and the concentration of the sample, namely the concentrations of IgM and IgG, was measured based on a concentration trend line obtained using the standard. In order to quantify the immunoglobulin ratio in the measured GCF, a specific ratio thereof was calculated(IgM/IgG), and the quantified ratio observed in a disease-specific manner was proposed as a biomarker.

### 1.3. Immunofluorescence Staining

Immunofluorescence staining used for visualizing the results of the present invention is a biological technique for visualizing an antigen in a specific cell or tissue by binding a fluorescent material to an antibody. The tissue to be analyzed was fixed in 10% formalin for 24 hours and then transferred to 80% ethanol. Thereafter, the fixed tissue was embedded using paraffin as a medium, sectioned to a thickness of 10 µm, and mounted on a slide. Thereafter, deparaffinization was performed in xylene three times for 5 minutes each, and dehydration was performed in 100% ethanol twice for 1 minute each. After rehydration was performed in distilled water twice for 1 minute each, antigen retrieval was performed for 10 minutes using boiled citrate buffer (#C9999, Sigma Aldrich, USA). After a permeabilization process was performed by treating the tissue with 0.1% Triton X-100 (#T8787, Sigma Aldrich, USA) diluted in DPBS for 10 minutes, incubation was performed for 1 hour using a 5% BSA solution diluted in DPBS in order to block nonspecific binding. Anti-CD138 (#ab128936, Abcam, USA) and anti-CD20 (#ab9475, Abcam, USA) were used as primary antibodies, diluted to a concentration of 2 µg/ml, and incubated overnight at 4°C in a state in which the tissue was immersed therein. Thereafter, the sample was washed with DPBS three times for 15 minutes each, and incubated with anti-rabbit (#A21203, Invitrogen, USA) and anti-mouse (#A11008, Invitrogen, USA) secondary antibodies (1:500) for 2 hours at room temperature in a light-shielded environment. Thereafter, nuclei were stained with DAPI (50 ng/ml) for 30 minutes, thereby completing immunofluorescence staining. Subsequently, immunofluorescence images were obtained using a confocal microscope (Carl Zeiss, Germany), and the visualized fluorescence information was quantified using ImageJ software (National Institutes of Health, USA). A "relative value of the number of fluorescently stained cells" was quantified and visualized by applying CD138 or CD20/DAPI.

### 1.4. Single-Cell Sequencing and Analysis

Gingival tissue used for single-cell RNA sequencing was first dissociated according to the manufacturer's instructions using Multi Tissue Dissociation Kit 2 (#130-110-203, Miltenyi Biotec, Germany). After dissociation, red blood cells were removed using Red Blood Cell Lysis Solution (#130-094-183, Miltenyi Biotec, Germany), and the cells were filtered through a 70 µm filter (#130-110-916, Miltenyi Biotec, Germany). The single-cell suspension was loaded onto a 10X Chromium Controller (10X Genomics, USA), and library preparation was performed according to the instructions of the 10X Chromium Next GEM Single Cell Library Kit v3.1 (#1000121, 10X Genomics, USA). The prepared library was sequenced using an Illumina HiSeq X sequencer (Illumina, USA) according to the read configuration recommended by 10X Genomics. The obtained sequencing data were analyzed using the Seurat R package (version 5.0.1).

### Example 2. Experimental Results

### 2.1. Results of Distribution of Periodontal Tissue-Specific Plasma Cells and B Cells in Patients with Aggressive Periodontitis Compared with Chronic Periodontitis

Cells expressing markers specific to plasma cells (CD138) and B cells (CD20) in periodontal tissues of patients with aggressive periodontitis and chronic periodontitis were visualized, whereby spatial information thereof could be specified, and the distribution of target cells(plasma cells and B cells) was quantified.

As a result of single-cell sequencing of periodontal tissues of patients with aggressive periodontitis and chronic periodontitis, a specific B-cell population increased in aggressive periodontitis could be identified.

As a result, in the periodontal tissue of patients with aggressive periodontitis, compared with patients with chronic periodontitis, the distribution of plasma cells, which are a developed form of B cells known to mainly secrete IgG, was found to be lower, whereas the distribution of B cells, which are a stage prior to development into plasma cells, was relatively high and a specific B-cell population was present. Accordingly, a basis supporting deficiency of a CSR process in B cells within periodontal tissue of patients with aggressive periodontitis was confirmed (FIG. 1).

**[Table 1]**

| Sociodemographic data for each patient used for analyzing GCF-specific immunoglobulin expression patterns and ratios thereof in patients with aggressive periodontitis. | | | | |
|---|---|---|---|---|
| Group | Age in years (mean ± SD) | Sex, n (%) | | n (Number of Replicates) |
| HC (Healthy) | 33.9 ± 15.10 | M (Male) | 2 (29.6) | 7 |
| | | F (Female) | 5 (71.4) | |
| CP (Chronic Periodontitis) | 62.8 ± 5.43 | M (Male) | 6 (42.9) | 14 |
| | | F (Female) | 8 (57.1) | |
| AP (Aggressive Periodontitis) | 43.5 ± 0.58 | M (Male) | 3 (50) | 6 |
| | | F (Female) | 3 (50) | |

The sociodemographic data of the patients used for quantifying immunoglobulins in GCF of each patient summarized in FIG. 2 were as shown in Table 1 above.

### 2.2. Results of Quantitative Analysis According to Enzyme-Linked Immunosorbent Assay (ELISA)

The ratio (IgM/IgG) of specific immunoglobulins and the IgM concentration in GCF for each patient used as each experimental group and control group were quantitatively analyzed using an Enzyme-Linked Immunosorbent Assay (ELISA), and the ratio thereof was quantified and shown in Table 2 below.

**[Table 2]**

| Group | HC | HC | HC | HC | HC | HC | HC | | | | | | | |
|---|---|---|---|---|---|---|---|---|---|---|---|---|---|---|
| Age in years | 25 | 32 | 23 | 26 | 31 | 67 | 33 | | | | | | | |

| Sex | F | F | M | M | F | F | F | | | | | | | |
|---|---|---|---|---|---|---|---|---|---|---|---|---|---|---|
| IgM/IgG (mean) | 0 135 | 0 342 | 0 168 | 0 077 | 0 094 | 0 031 | 0 020 | | | | | | | |

| Group | CP | CP | CP | CP | CP | CP | CP | CP | CP | CP | CP | CP | CP | CP |
|---|---|---|---|---|---|---|---|---|---|---|---|---|---|---|
| Age in years | 66 | 63 | 55 | 67 | 57 | 35 | 62 | 26 | 37 | 54 | 72 | 23 | 62 | 68 |

| Sex | F | F | F | M | M | M | M | F | F | F | M | F | M | F |
|---|---|---|---|---|---|---|---|---|---|---|---|---|---|---|
| IgM/IgG (mean) | 0 196 | 0 040 | 0 667 | 0 336 | 0 007 | 0 012 | 0 047 | 0 026 | 0 037 | 0 061 | 0 030 | 0 030 | 0 047 | 0 037 |

| Group | AP | AP | AP | AP | AP | AP | | | | | | | | |
|---|---|---|---|---|---|---|---|---|---|---|---|---|---|---|
| Age in years | 43 | 44 | 43 | 44 | 39 | 34 | | | | | | | | |

| Sex | M | F | M | M | F | F | | | | | | | | |
|---|---|---|---|---|---|---|---|---|---|---|---|---|---|---|
| IgM/IgG (mean) | 0 846 | 1 665 | 2 166 | 0 226 | 0 313 | 0 371 | | | | | | | | |

The ratio (IgM/IgG) of specific immunoglobulins in GCF of each patient group used in the ELISA analysis of FIG. 2.

The ratio of specific immunoglobulins (IgM and IgG) and the IgM concentration in GCF of patients with aggressive periodontitis, patients with chronic periodontitis, and a control group were quantified, statistical significance thereof was verified, and the results were shown in graphs. As a result of quantifying the immunoglobulin concentration in each experimental group and control group, it was found that the concentration of IgM relative to IgG was disease-specifically high in GCF of patients with aggressive periodontitis, and it was confirmed that this was independent of the concentration of IgG. This was numerically represented as the ratio of IgM relative to IgG in GCF and the absolute concentration of IgM. It was confirmed that the numerical values have high applicability as biomarkers for predicting progression of aggressive periodontitis, diagnosing aggressive periodontitis, discriminating a high-risk group, and exploring applicability as a future therapeutic strategy (FIG. 2).

Next, at an endpoint of a 5-year retrospective cohort, GCF was collected according to the grade and stage of periodontitis, and the IgM/IgG ratio and the IgM concentration were measured. It was confirmed that the IgM/IgG ratio and the IgM concentration tended to increase as the stage and grade of periodontitis became more severe, suggesting that the IgM/IgG ratio and the IgM concentration are closely associated with disease severity (FIG. 3).

When the results of FIGS. 2 and 3 are described in combination with the results of FIG. 1, in the case of patients with aggressive periodontitis, it can be explained that, due to suppression of CSR function, appropriate production of immunoglobulins does not occur in the physiological environment thereof, thereby causing a disease-specific immunoglobulin expression pattern. This suggests that, due to a problem in the immune system caused by suppression of CSR function, production of immunoglobulins is not properly regulated specifically in patients with aggressive periodontitis.

Consequently, a specific immunoglobulin secretion and B-cell development pattern in which the ratio of IgM is significantly increased in GCF of patients with aggressive periodontitis was derived, and through this, usefulness as a biomarker capable of helping determine or predict aggressiveness of periodontitis according to the IgM expression level was suggested.

Although specific portions of the present invention have been described in detail above, it will be apparent to those of ordinary skill in the art that such specific descriptions are merely preferred embodiments and that the scope of the present invention is not limited thereby. That is, the substantial scope of the present invention is defined by the appended claims and equivalents thereof.

The national research and development project supporting the present invention is as follows.

[Project Identification Number] 2710012269
[Project Number] 2022M3A9F3016364
[Ministry] Ministry of Science and ICT
[Project Management / Specialized Agency] National Research Foundation of Korea
[Research Program Name] Bio & Medical Technology Development Program
[Research Project Name] an oral microbiome function evaluation platform and source technology for disease control
technology development
[Institution Performing the Project] Yonsei University
[Research Period] April 1, 2022 to December 31, 2026

## Claims

1. A method for providing information on diagnosis or prognostic evaluation of periodontitis, comprising:
measuring information indicating a level of B cells at a stage before being developed into plasma cells in a biological sample isolated from a subject; and
providing information on diagnosis or prognostic evaluation of periodontitis from the information.

2. The method of claim 1,
wherein the information indicating the level of B cells at a stage before being developed into plasma cells in the biological sample isolated from the subject comprises an IgM expression level in the biological sample.

3. The method of claim 1,
wherein the biological sample is derived from gingival crevicular fluid of the subject.

4. The method of claim 1,
wherein the periodontitis is aggressive periodontitis.

5. The method of claim 1,
wherein the periodontitis is chronic periodontitis.

6. The method of claim 1,
wherein the periodontitis is Grade C periodontitis.

7. The method of claim 1,
wherein the periodontitis is Grade B periodontitis.

8. The method of claim 1,
wherein the periodontitis is Grade A periodontitis.

9. The method of claim 1,
wherein the level of B cells at a stage before being developed into plasma cells in the biological sample isolated from the subject is higher than a reference level indicates one or more of the following states:
i) the subject has a rapid progression rate of periodontitis or has severe periodontitis;
ii) the subject has a high risk of being diagnosed with Grade B periodontitis, Grade C periodontitis, or aggressive periodontitis;
iii) the subject has a high risk of being diagnosed with stage 3 or stage 4 periodontitis; and
iv) the subject has a high risk of being diagnosed with IgM-secreting B cell-infiltrated periodontitis.

10. The method of claim 1,
wherein the level of B cells at a stage before being developed into plasma cells in the biological sample isolated from the subject is lower than a reference level indicates one or more of the following states:
i) the subject does not have a rapid progression rate of periodontitis and does not have severe periodontitis;
ii) the subject has a low risk of being diagnosed with Grade B periodontitis, Grade C periodontitis, or aggressive periodontitis;
iii) the subject has a low risk of being diagnosed with stage 3 or stage 4 periodontitis; and
iv) the subject has a low risk of being diagnosed with IgM-secreting B cell-infiltrated periodontitis.

11. The method of claim 2,
wherein the IgM expression level in the biological sample isolated from the subject is higher than a reference level indicates one or more of the following states:
i) the subject has a rapid progression rate of periodontitis or has severe periodontitis;
ii) the subject has a high risk of being diagnosed with Grade B periodontitis, Grade C periodontitis, or aggressive periodontitis;
iii) the subject has a high risk of being diagnosed with stage 3 or stage 4 periodontitis; and
iv) the subject has a high risk of being diagnosed with IgM-secreting B cell-infiltrated periodontitis.

12. The method of claim 2,
wherein the IgM expression level in the biological sample isolated from the subject is lower than a reference level indicates one or more of the following states:
i) the subject does not have a rapid progression rate of periodontitis and does not have severe periodontitis;
ii) the subject has a low risk of being diagnosed with Grade B periodontitis, Grade C periodontitis, or aggressive periodontitis;
iii) the subject has a low risk of being diagnosed with stage 3 or stage 4 periodontitis; and
iv) the subject has a low risk of being diagnosed with IgM-secreting B cell-infiltrated periodontitis.

13. The method of any one of claims 9 to 12,
wherein the reference level is equivalent to any one measured as follows:
i) measured from a biological sample isolated from a normal subject;
ii) measured from a biological sample isolated from a patient suffering from Grade A periodontitis; or
iii) measured from a biological sample isolated from a patient suffering from Grade B periodontitis.

14. The method of claim 2,
wherein a method for measuring the expression level of IgM is selected from the group consisting of Western blotting, immunochemical analysis, flow cytometry, and a protein chip.

15. The method of claim 1,
wherein the information on diagnosis or prognostic evaluation of periodontitis is information on a grade, stage, severity and/or aggressiveness of periodontitis.

16. A biomarker composition for diagnosing or evaluating a prognosis of periodontitis, comprising an agent capable of measuring an IgM expression level in a biological sample isolated from a subject.

17. The biomarker composition of claim 16,
wherein the agent capable of measuring the IgM expression level is one or more selected from the group consisting of an antibody, a peptide, an aptamer, and a compound that specifically bind to IgM.

18. The biomarker composition of claim 16,
wherein the biological sample is derived from gingival crevicular fluid of the subject.

19. A kit comprising the biomarker composition of claim 16.

20. The kit of claim 19,
wherein the kit is for providing information on a grade, stage, severity and/or aggressiveness of periodontitis.
